# EUROPEAN PATENT APPLICATION

(11) **EP 1 543 847 A1**
(43) Date of publication of application: **22.06.2005**
(21) Application number: 04380248.7
(22) Date of filing: 02.12.2004
(51) Int. Cl.: A61L 15/46

(54) **Use of chlorophyll for controlling unpleasant odors in disposable absorbent articles**

(30) Priority: 19.12.2003 MX pa03000086
(71) Applicant: Grupo P.I. Mabe, S.A. de C.V., 72230 Puebla, Estado de Puebla (MX)
(72) Inventor: Corona Carlos, Alberto, Col. La Loma, 72230 Puebla (MX); Canales Espinosa De Los Monteros, Carlos, Col. La Loma, 72230 Puebla (MX); Sanchez Fernandez, Lucia, Col. La Loma, 72230 Puebla (MX); Fajardo Eslava, Bernardo, Col. La Loma, 72230 Puebla (MX)
(74) Representative: Gil-Vega, Victor

(57) **Abstract**

The present invention discloses the use of chlorophyll or any of its derivatives, a natural-origin compound, to help reduce unpleasant odors during the use of disposable absorbent articles. The chlorophyll can be applied in the absorbent core of the article or over the topsheet of same.

## Description

### Background of the Invention

Disposable absorbent articles, such as disposable diapers for babies, disposable diapers for adults, training pants, disposable articles for incontinence, sanitary napkins, panty-liners and other similar articles, are articles intended for absorbing and containing body exudates and to be discarded after their use. There are many proposals focusing on improving the characteristics and features of this type of products, some focusing on improving absorption and retention, others focusing on preventing leakages, some others more to make the articles soft and comfortable for the user as well as discreet, others to reduce costs, etc.

Another research line to improve this type of articles is directed to eliminating undesirable odors during their use, such as odors caused by body exudates and decomposition of same. Some of the patents in the literature in regard to this topic are US 5,932,495, in the name of Kimberly-Clark, which discloses the use, on a substrate such as a film or a fabric, of a chitosan or alginate in combination with a triglyceride for the absorption of unpleasant odors in disposable absorbent articles, where the triglyceride has the function of increasing the efficacy of chitosan in at least about 50%; US 6,417,424, in the name of Procter & Gamble, describes a breathable, disposable absorbent article containing silica, zeolites, activated carbon or mixtures thereof in the absorbent core in order to control unpleasant odors; WO 00/67688 A1, in the name of Capitol Specialty Plastics, disclosed the application of a liquid for absorbing odors which is made by means of the reaction of a natural organic acid and glycerine, over a film such as a non-woven fabric, to be used in a disposable absorbent article as the topsheet thereof; EP 1 149 594 A1, in the name of Procter & Gamble, discloses a disposable absorbent article containing in its absorbent core a mixture of chitosan and an anionic absorbent gel to help eliminate unpleasant odors, also increasing the absorption properties of the article; US 6,225,524, also in the name of Procter & Gamble, describes the use of silica or a metallic silicate in combination with an absorbent gel in the absorbent core of the article in order to control unpleasant odors.

As can be seen from the preceding paragraph, there is a great deal of proposals directed to controlling unpleasant odors in disposable absorbent articles; however, none of them contemplate the use of a natural compound, such as chlorophyll, for this purpose.

Chlorophyll has been used in some foodstuffs and cosmetics as a dye; on the other hand, *in vitro* experiments have shown its efficacy in neutralizing unpleasant odors, and has been used for this purpose in human beings for neutralizing unpleasant odors caused by consumption of foods, beverages, tobacco, etc., as well as for neutralizing unpleasant odors caused by sweating.

Chlorophyll, in its natural state, has in the center of its structure a magnesium molecule, which contains small toxic portions for human consumption and in insoluble in water; however, upon replacing the magnesium molecule with a copper, sodium or potassium molecule by means of ionic exchange, non-toxic and water soluble chlorophyll derivatives are obtained, such as cupric chlorophyll, the use of which has been approved in the United States of America by the Food and Drug Administration and the Toxic Substances Control Act. Due to its being a natural-origin deodorant, chlorophyll is biodegradable, degrading in carbon dioxide, purine, cupric oxide and some unidentified compounds.

The present invention proposes the use of chlorophyll or its derivatives in disposable absorbent articles to help control unpleasant odors caused by body exudates, in view of the advantages it shows due to its being a biodegradable, natural product and its use being accepted by national and international organisms. Chlorophyll can be applied directly on the absorbent core of the article, mixed within same or, properly diluted, applied over the topsheet of the article.

On the other hand, also described is the use in this type of articles of a mixture of chlorophyll with some commonly used agent for odor control, such as silicon containing compounds, compounds made from soy ethoxylate or other compounds known in this field for this purpose, since tests of the product have shown that, when chlorophyll is mixed with this type of products, there is a synergistic effect between both such that they work more efficiently in combination.

### Objects of the Invention

An object of the present invention consists of the use of a natural product to help reduce unpleasant odors caused by body exudates in disposable absorbent articles.

A further object of the invention consists of the use of a compound for controlling unpleasant odors that is innocuous, does not provoke negative skin reactions, and totally or partly of a natural origin.

An additional object of the invention consists of using a compound that can be easily applied in a disposable absorbent article.

### Detailed Description of the Invention

Disposable absorbent articles are articles which are used in contact with the body to receive and contain body exudates; among them, disposable diapers for babies, training pants, sanitary napkins, panty-lines and articles for incontinence can be mentioned. Body exudates, such as urine, feces and menstrual fluids have by themselves an unpleasant odor, and they can in addition decompose into other compounds which also posses unpleasant odors; for this reason, it has become a common practice in industry to incorporate in disposable absorbent articles some compound or product which helps to reduce unpleasant odors.

Chlorophyll is a natural-origin deodorant; experiments performed in human beings have shown its efficacy in neutralizing unpleasant odors caused by the consumption of foods, beverages, tobacco, etc., due to sweating, metabolic changes, and menstruation. However, it has never been used in disposable absorbent articles; as a result, the present invention proposes the use of chlorophyll or its derivatives, which are compounds of a natural origin, to help neutralize unpleasant odors originated from body exudates in disposable absorbent articles. Chlorophyll is a natural adsorbent which, by means of Van der Waals forces, attracts the unpleasant odor-generating compounds, which adhere to the chlorophyll molecule, becoming trapped therein.

In this type of articles, chlorophyll or its derivatives can be used in powder form, mixed with the absorbent material or located in specific points within the absorbent core of the article, or can be used in an aqueous solution applied wither over the absorbent core of the article or over the topsheet thereof.

When used in powder form, chlorophyll has shown its efficacy when employed in amounts from about 0.01% by weight, that is when using from about 0.0001 grams of chlorophyll per gram of absorbent material, either uniformly mixed with the absorbent material or located in specific points thereof. It being understood that the use of any of the chlorophyll derivatives provides the same efficiency in regard to the elimination of odors. Chlorophyll, as such, contains some portions that may be toxic for human consumption or use; however, it does not cause any adverse reaction in contact with the skin, for which reason chlorophyll or any of its derivatives can be used mixed with the absorbent material of the article; in addition, the chlorophyll would never be in direct contact with the skin, since over the absorbent core is the topsheet of the article, which is the one in contact with the skin.

In order to use chlorophyll in an aqueous solution, either over the absorbent core or over the topsheet of the article, it is necessary to exchange the magnesium contained therein with another metal capable of ionization which makes it soluble, such as potassium, sodium or copper, the latter being the most used, since cupric chlorophyll is accepted for use and for human consumption by the Food and Drug Administration and the Toxic Substance Control Act in the United States of America, as previously mentioned.

When used in an aqueous solution, cupric chlorophyll or any other derivative of same with water-solubility properties, is applied over the absorbent core of the article or over the topsheet of same, assuring a minimum application of about 0.01 grams of chlorophyll per square meter.

In practice, it is more convenient to dissolve the chlorophyll derivatives with solubility properties in any type of vehicle different from water to prevent growth of microorganisms; as an example, pharmaceutical grade propylene glycols (USP) can be used for dissolving chlorophyll, or any other solution vehicles known by those skilled in the art.

Consumer panel tests have shown the effectivity of the use of chlorophyll or its derivatives to control unpleasant odors in this type of articles. As an example, cupric chlorophyll was applied in a 1.5% solution in monopropylene glycol over non-woven fabric used in the manufacture of sanitary napkins, assuring an amount of about 0.03 grams of cupric chlorophyll by square meter of non-woven fabric. The sanitary napkins were tested by a panel of 50 consumers; the results show that 85% of the users noticed a reduction in the characteristic odor of menstrual fluids when using the napkin. In tests with disposable diapers, applying the same amount of chlorophyll to the non-woven fabric used as topsheet of the article, the results were of 88% of the persons changing the disposable diapers to users noticed a reduction in the unpleasant odors caused by urine and feces.

In order to increase the effectivity of chlorophyll, same can be used in combination with other odor-control agents, such as silicon- and zinc-containing compounds which rapidly and efficiently attract and trap unpleasant odors, making them non volatile. As an example of these products, Syngard, made by Corporación Industrial Staquim, S.A. de C.V., can be used, or compounds made from soy ethoxylate, which has the capability of neutralizing unpleasant odors from organic molecules; an example of this type of compounds is Forestall, from Uniquema, or any other compound or element used for this purpose, such as silica or a zeolite.

While the invention has been described as a function of a currently preferred embodiment, it is apparent that changes and variations thereof can be made, all such changes and variations falling with the scope and spirt of the invention, as defined in the appended claims.

## Claims

1. A disposable absorbent article, basically comprising a topsheet, a backsheet, and an absorbent core disposed between them, **characterized in that** it contains chlorophyll or a derivative thereof, to reduce unpleasant odors caused by body exudates.

2. A disposable absorbent article, as recited in claim 1, **characterized in that** the chlorophyll or its derivatives are disposed in the absorbent core of the article.

3. A disposable absorbent article, as recited in claim 2, **characterized in that** the chlorophyll or its derivatives are used in powder form, mixed with the absorbent material of the absorbent core of the article, in a minimum amount of about 0.0001 grams or chlorophyll or its derivatives per gram of absorbent material.

4. A disposable absorbent article as recited in claim 1, **characterized in that** a solution of chlorophyll or its derivatives is used, applied over the absorbent core the article such that the minimum application is of about 0.01 grams of chlorophyll or its derivatives per square meter of absorbent material.

5. A disposable absorbent article, as recited in claim 1, **characterized in that** a solution of chlorophyll or its derivatives is used, applied over the film used as topsheet of the article such that the minimum application is of about 0.01 grams of chlorophyll per square meter of film.

6. A disposable absorbent article, as recited in claims 4 and 5, **characterized in that** the chlorophyll solution further contains one or more pharmaceutical-grade solution vehicles.

7. A disposable absorbent article, as recited in any of the preceding claims, **characterized in that** chlorophyll or its derivatives are used in a mixture with a silicon- or zinc-based mixture to control unpleasant odors.

8. A disposable absorbent article, as recited in any of claims 1 through 6, **characterized in that** chlorophyll or its derivatives are used in a mixture with a soy ethoxylate-based compound to control unpleasant odors.

9. A disposable absorbent article, as recited in any of claims 1 through 6, **characterized in that** chlorophyll or its derivatives are used in a mixture with silica to control unpleasant odors.

10. A disposable absorbent article, as recited in any of claims 1 through 6, **characterized in that** chlorophyll or its derivatives are used in a mixture with a zeolite to control unpleasant odors.

11. A disposable absorbent article, as recited in any of the preceding claims, **characterized in that** the chlorophyll is a cupric chlorophyll as a chlorophyll derivative to help control unpleasant odors.
